(19) Europäisches Patentamt / European Patent Office / Office européen des brevets

(11) **EP 2 311 975 A1**

(12) **EUROPEAN PATENT APPLICATION**

(43) Date of publication:
20.04.2011 Bulletin 2011/16

(51) Int Cl.:
*C12Q 1/68* (2006.01)  *G01N 27/447* (2006.01)
*G01N 33/487* (2006.01)

(21) Application number: 09172950.9

(22) Date of filing: 13.10.2009

(84) Designated Contracting States:
AT BE BG CH CY CZ DE DK EE ES FI FR GB GR HR HU IE IS IT LI LT LU LV MC MK MT NL NO PL PT RO SE SI SK SM TR
Designated Extension States:
AL BA RS

(71) Applicant: **IEE INTERNATIONAL ELECTRONICS & ENGINEERING S.A.**
**6468 Echternach (LU)**

(72) Inventor: **Boulbitch, Alexei**
**54295 Trier (DE)**

(74) Representative: **Office Freylinger**
**P.O. Box 48**
**8001 Strassen (LU)**

(54) **Electrophoretic biomolecules separation device**

(57) A device for electrophoretic separation of biomolecules comprises a vessel with an inlet chamber (20), a drift chamber (22) and an outlet chamber (24), a first partition (26) that separates the inlet chamber (20) from the drift chamber (22) and a second partition (28) that separates the drift chamber (22) from the outlet chamber (24). The first partition has therein at least one nanopore (30) allowing fluid communication and passage of biomolecules between the inlet chamber and the drift chamber. The second partition has therein at least one nanopore (32) allowing fluid communication and passage of biomolecules between the drift chamber and the outlet chamber. The device further comprises a first and a second electrode (34,36) associated with the inlet chamber (20) and the outlet (24) chamber, respectively, for generation of an ionic current through the nanopores and the drift chamber.

**Fig. 7**

## Description

## Technical field

The present invention relates to electrophoretic separation of biological molecules (hereinafter "biomolecules"), such as, e.g., proteins, polysaccharides, DNA or RNA fragments, in particular to a device for electrophoretic separation of biomolecules.

## Background Art

Several approaches to separate the different species of a mixture of molecules and to identify the species based upon that separation are known in the state of the art.

Some methods are based on gas-phase time-of-flight measurements. In ion-mobility spectroscopy [1], the analyte molecules are ionized in gas phase and the mixture of ions is injected into an analyzing chamber, wherein the ions are carried by electric field towards registration electrodes. Due to different mobilities of different ion species, the latter are separated into swarms (clouds) that are detected at the electrodes at different points of time. The time of flight serves here as the fingerprint of the ion species. The method works well with small organic molecules, such as, e.g. benzene derivatives, hydrocarbons and molecules of explosives, like trinitrotoluene. The method fails, however, in effectively separation of large biological molecules, such as proteins. This is due to the complex and poorly understood gas-phase chemistry of biomolecules. Another problem arises from the fact that biomolecules are inevitably denatured in gas phase and may decay or form parasitic complexes. All this results in ion-mobility spectroscopy being currently unacceptable for reliable analysis of biomolecules, in particular proteins.

Time-of-flight mass-spectrometry [2, 3] is considered to be one of the most effective methods to identify molecules, since it enables one to measure the charge-to-mass ratio of the analyte. If dealing with relatively small molecules, one is usually able to unambiguously identify the species of the analyte based on the charge-to-mass ration. With large molecules, such as proteins, the number of mass and charge number combinations yielding the same charge-to-mass ratio becomes so great that determination of a single matching combination and thus, identification of the molecule species becomes impossible. Furthermore, time-of-flight mass spectroscopy shares the drawbacks of ion mobility spectroscopy as it is carried out in gas phase.

The most widely used method for separation and identification of biomolecules is referred to as gel-electrophoresis. In this method, the mixture of biomolecules is forced by an electric field to drift through a gel. The time of drift is used as a fingerprint of the biomolecules. This method is presently used as a basis for detection and identification of proteins. Since the parameters of the gel are critical for the detection, the main drawback of this method is the requirement to prepare a new gel matrix for each measurement, the preparation conditions being strictly identical to one another. Another drawback is the long duration of a single measurement, which may span from over several hours to over a few days. Finally, in order to visualize the biomolecules after the separation, they are typically chemically modified before the separation using fluorescent labels, and irradiated with UV light after the separation. This requires a bulky and expensive optical instrumentation, which represents another drawback of this approach.

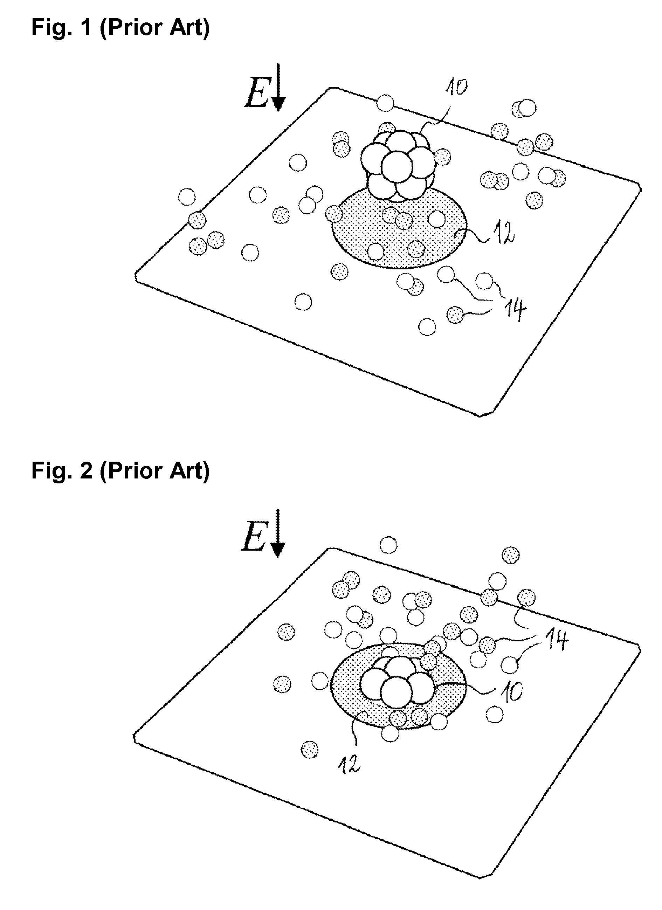

Recently, several attempts to identify biomolecules using solid-state nanopores have been reported [4-8]. Solid-state nanopores are small through-holes in thin solid plates (with thickness of below 100 nm in the area around the nanopore) bored using an ion or/and electron beam, or ion beam assisted etching [4]. The diameter of the pore can be achieved to be as small as 2 nm [4], but typical values are about 10 to 20 nm [4, 5]. The nanopores have been used to try to distinguish DNA [4, 6-8] as well as proteins [5]. The method of detection consisted in using a vessel divided into two parts by a plate comprising the nanopore as the sole channel between the two parts of the vessel. The vessel is filled with electrolyte solution, and DNA molecules or proteins are introduced into the first part of the vessel (referred to as the "cis" part). As illustrated in Figs. 1 and 2, application of an electric voltage across the vessel causes the charged biomolecules 10 to move towards the nanopore 12 and then through the nanopore 12 into the second part (the "trans" part) of the vessel (Fig. 1). During its motion through the nanopore (Fig. 2), 12, a biomolecule 10 blocks a considerable part of the cross sectional area of the nanopore 12 which gives rise to a manifest drop of the ionic current caused by the movement of small ions 14 of the electrolyte solution. The current flowing across the vessel can be recorded by a straightforward electrical measurement.

The papers [4-8] report on experiments to distinguish DNA molecules or proteins based on the characteristics of these drops in ionic current. Up to now, however, these attempts have been unsuccessful. The main reason for this is the high speed of motion of the molecules through the nanopore. For example, it takes about 0.1 $\mu$s for a DNA base pair [4] and from a few microseconds to a fraction of a millisecond for proteins [5] to pass through the nanopore. These times are too small to characterize the species of the molecules passing through the nanopore. Furthermore, the amplitude of the current drop and the duration thereof represent only two parameters, which do not seem to qualify as fingerprints of the different molecule species.

In the following, the term "electrophoresis" designates the motion of dispersed particles relative to a fluid under the influence of an electric field. Accordingly, "electrophoretic separation" designates a process, by which molecules are separated according to size and electrical charge by applying an electric current through the medium in which these molecules are dispersed.

## Technical problem

It is an object of the present invention to improve electrophoretic separation of biological molecules. This is achieved using a device as claimed in claim 1.

## General Description of the Invention

According to the invention, a device for electrophoretic separation of biomolecules comprises a vessel with an inlet chamber, a drift chamber and an outlet chamber, a first partition that separates the inlet chamber from the drift chamber and a second partition that separates the drift chamber from the outlet chamber. The first partition has therein at least one nanopore allowing fluid communication (i.e. passage of liquid or gas) and passage of biomolecules between the inlet chamber and the drift chamber. The second partition has therein at least one nanopore allowing fluid communication and passage of biomolecules between the drift chamber and the outlet chamber. The device further comprises a first and a second electrode associated with the inlet chamber and the outlet chamber, respectively, for generation of an ionic current through the nanopores and the drift chamber. Except for the nanopores, the first and second partitions are fluid-tight, whereby it is guaranteed that an ionic current through the first or second partition can only flow through the nanopores in the first and second partition, respectively.

The device according to the invention may be used for analyzing a sample containing biomolecules by filling the chambers of the device with an electrolyte solution compatible with the biomolecules of the sample, introducing the sample into the inlet chamber and applying a voltage across the electrodes. The voltage causes an ionic current to flow through the nanopores and the drift chamber. Under the action of the electric field, the biomolecules are caused to migrate from the inlet chamber into the drift chamber through the at least one nanopore of the first partition, to move across the drift chamber at a drift rate in accordance with their size-to-charge relation, and to migrate from the drift chamber into the outlet chamber through the at least one nanopore of the second partition. It should be noted that the electric field is typically inhomogeneous - and hence the drift rate is not constant. While the voltage is applied, one records the resistance and/or the current flowing between the first and second electrodes as a function of time. Each time a biomolecule passes through a nanopore in the first or the second partition, it partially obstructs the nanopore and reduces the free cross section thereof, which is available for small ions ($Na^+$, $K^+$, $Cl^-$, or whichever other small ions might be present in the solution) to pass from one side of the partition to the other. Accordingly, each time a biomolecule "translocates" (see [4]) through a nanopore, the electrical resistance between the first and second electrode increases (and the current drops). When the electric field is applied, the biomolecules pass through the nanopore of the first partition (the first partition may comprise a single nanopore or a plurality thereof) in an unordered manner, causing a series of current drops that are close to one another in time. In the drift chamber, molecules of different species move towards the at least one nanopore of the second partition (the second partition may also comprise a single nanopore or a plurality thereof) at different drift rates. Indeed, the drift rate of each species depends on the size-to-charge ratio of the molecules of that species. As the molecules migrate through the drift chamber, the different species thus separate into different swarms or clouds, which arrive at the nanopore of the second partition at different times, without there being an overlap of swarms if the device is properly dimensioned. Each swarm thus causes a series of current drops as its molecules pass through the nanopore of the second partition. As each swarm arrives at a different time, the different series of current drops show no overlap along the time axis and may be distinguished from one another. The time of drift of each species may be determined as the time interval between the first current drop (caused by the passage of the biomolecules through the nanopore of the first partition) and the later current drop (caused by the biomolecules of the particular species through the nanopore of the second partition). The time-of-drift may be used as a fingerprint of the biomolecules of the sample. Identification of the different species may thus be achieved by comparison of the measured times-of drift and stored reference values. It may, in addition, even be possible to draw conclusions as to the relative amounts of the different species within the sample under examination by comparing the durations of the different series of current drops.

A benefit of the device according to the invention is that the initial current drop may be taken as the event that "starts the clock" for the time of drift measurement. This event can be clearly identified in the recorded data, which facilitates manipulation. The device furthermore facilitates ensuring the reproducibility of experiments, since at the point in time, from which the times of drift are computed in each experiment, the location of the biomolecules is the same for each experiment carried out with a given device.

As those skilled will appreciate, the device according to the invention has the potential to considerably reduce the time necessary for analyzing a sample in comparison with gel-electrophoresis.

Preferably the nanopores have a diameter adapted to the size of the biomolecules in such a way that the free cross section area of the nanopores is substantially reduced, e.g. by at least 20%, more preferably by at least 30% and yet more preferably by at least 50%, when the biomolecules pass there through. As typical effective diameters of biomolecules are typically contained in the range from 10 to 25 nm, the cross section diameter of the nanopores is preferably selected in the range from 2 to 50 nm, preferably in the range from 10 to 30 nm.

The cross section diameter of the at least one nanopore in the first partition is preferably selected in the range from of 1 to 4 times, more preferably in the range of 1 to 3 times, still more preferably in the range of 1 to 2 times, the cross section diameter of the at least one nanopore in the second partition. It may be preferable that the diameter of the nanopores in the first partition is greater than that of the nanopores of the second partition to allow biomolecules to pass through the first partition more quickly (i.e. within a shorter time interval) and thereby enable a more precise determination of the starting point of the drift interval. Alternatively, the cross section diameter of the at least one nanopore of the first partition could be equal to the cross section diameter of the at least one nanopore of the second partition. To ascertain a quick passage of the biomolecules through the first and/or the second partition under the action of the electric field, the number of nanopores in each partition can be adjusted.

The channel length of the nanopores (i.e. the thickness of the partition in the region surrounding each nanopore) is preferably of the same order of magnitude as the cross section diameter, e.g. comprised in the range from 0.5 to 10 times the cross section diameter of the nanopores.

The first and/or the second partition preferably comprises a membrane, e.g. a solid-state membrane, in which the at least one nanopore is arranged.

From the above explanations it follows that it may be advantageous to keep the biomolecules of the sample relatively close together when they are to pass across the first partition (to avoid too much spreading of the first series of current drops over time). Therefore, according to a preferred embodiment of the device, the volume of the inlet chamber is smaller, preferably at least 5 to 10 times smaller, than the volume of the drift chamber. More preferably, the ratio of inlet chamber volume to drift chamber volume is less than 1/100 or even less than 1/1000.

The device preferably comprises an injection system (e.g. an injection nozzle) for injecting a sample containing biomolecules into the inlet chamber.

The chambers preferably contain an electrolyte solution adapted to the biomolecules to be analysed, such as, e.g. normal saline (also referred to as physiological saline or isotonic saline). The internal surfaces of the chambers of the device, including the surfaces of the partitions, are preferably passivated. Passivation could e.g. be achieved by incubation of the surfaces with bovine serum albumine, followed by rinsing them with water. Any surface passivation technique that renders the internal surfaces of the device compatible with the biomolecules to be analysed may be used.

According to a preferred embodiment of the invention, the partitions are arranged substantially parallel to one another. The at least one nanopore of the first partition is preferably arranged in facing relationship with the at least one nanopore of the second partition. Most preferably, the nanopores are arranged approximately on the intersection of the partitions with a straight line extending from the first to the second electrode. However, other geometries can be used, since they can readily be calibrated with respect to a reference geometry via measurements with known samples.

Preferably, the device is equipped with a measurement circuit for applying a voltage across the first and second electrodes and to record resistance and/or current between the first and second electrodes as a function of time.

The drift chamber could optionally be subdivided into two or more sections by means of one or more further partitions, each of these further partitions comprising at least one nanopore allowing fluid communication and passage of biomolecules between neighboring sections. The lengths of the drift chamber sections in the direction of the current flow are preferably chosen in such a way that each series of current drops can be associated with passage of a biomolecule species through a particular partition - in other words, current drops caused by passage of biomolecules through one partition should not overlap in time with current drops caused by passage of quicker or slower biomolecules through another partition.

**Brief Description of the Drawings**

A preferred embodiment of the invention will now be described, by way of example, with reference to the accompanying drawings in which:

Fig. 1    (already addressed in the introduction) is a perspective schematic drawing of a biomolecule immersed in an electrolyte solution when approaching a nanopore under the action of an electric field;

Fig. 2    (already addressed in the introduction) is a perspective schematic drawing of the biomolecule of Fig. 1 as it passes through the nanopore and thereby reduces the cross section available for the passage of small ions;

Fig. 3    is a schematic drawing of an electrophoretic biomolecules separation device according to a preferred embodiment of the invention;

Fig. 4    is an enlarged view of detail II of Fig. 3;

Fig. 5    is a schematic drawing of the device of Fig. 3 illustrating the passage of a mixture of biomolecules through the first partition;

Fig. 6    is a schematic drawing of the device of Fig. 3 illustrating the separation of biomolecules species in the drift chamber;

Fig. 7   is a schematic drawing of the device of Fig. 3 illustrating the passage of the quickest biomolecule species through the second partition;

Fig. 8   is a graph illustrating the behaviour of ionic current as a function of time during a separation experiment;

Fig. 9   is a graph is a graph representing the time of drift of a typical biomolecule as a function of the drift distance;

Fig. 10   is a graph illustrating the impact of the difference in size of the biomolecule on the difference in times of drift;

Fig. 11   is a schematic drawing of an electrophoretic biomolecules separation device according to a second preferred embodiment of the invention;

Fig. 12   is a 3D schematic view of detail XII of Fig. 11.

## Description of a Preferred Embodiment

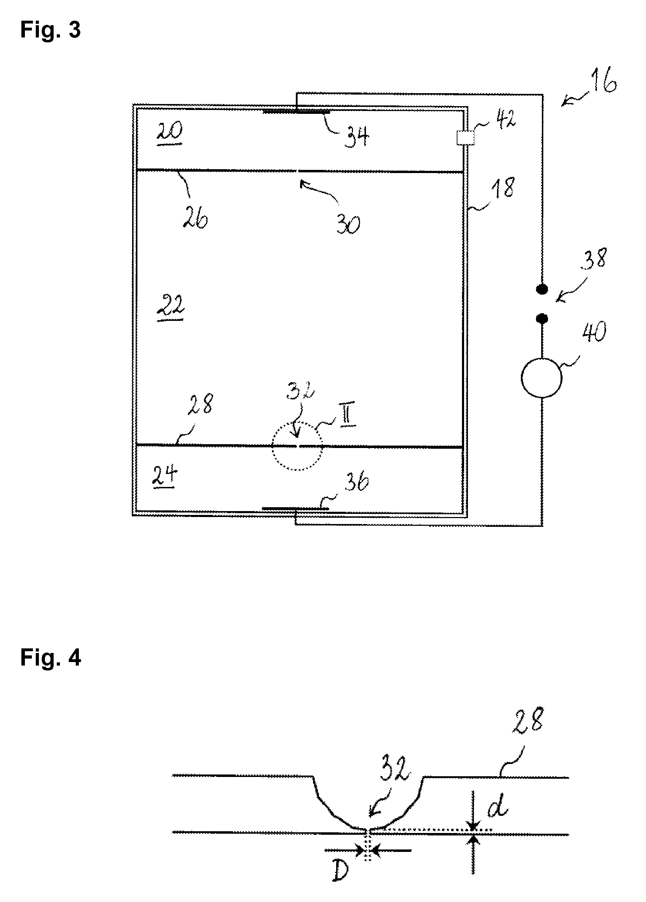

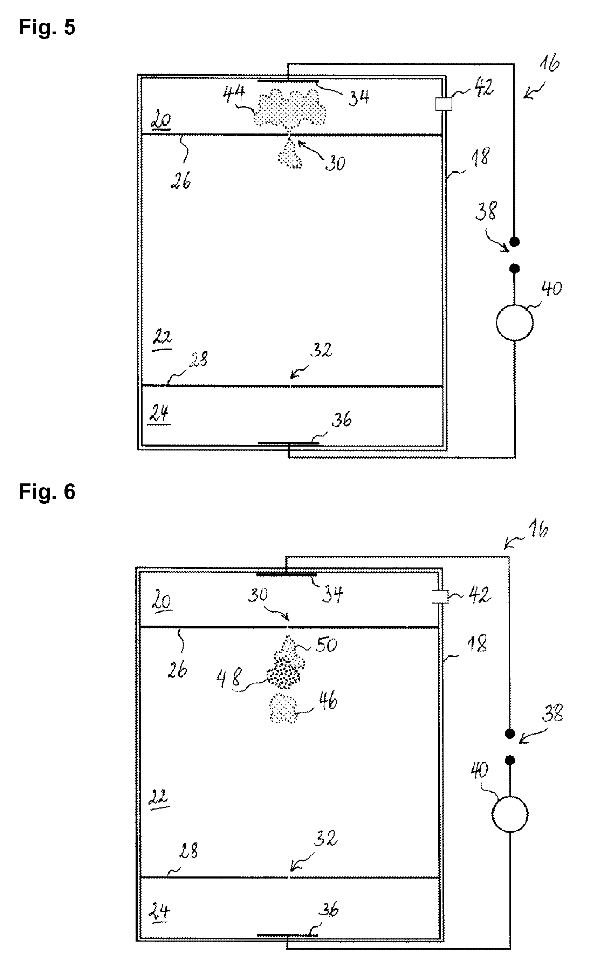

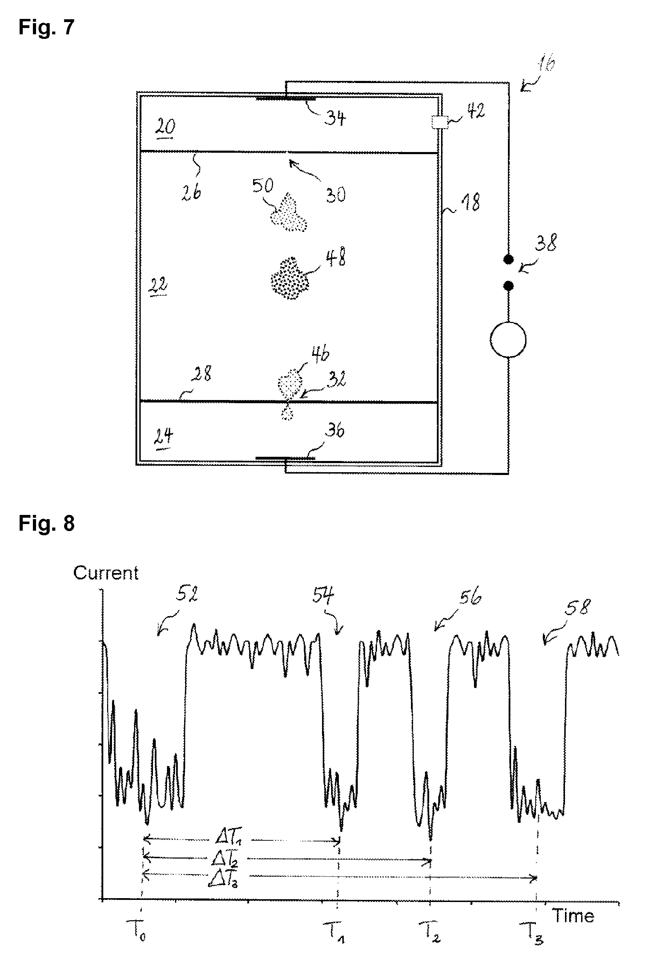

A preferred embodiment of an electrophoretic biomolecules separation device according to the invention, as well as operation of such a device is now explained with reference to Figs. 3-8. The electrophoretic biomolecules separation device 16 comprises a receptacle 18, which is subdivided into an inlet chamber 20, a drift chamber 22 and an outlet chamber 24. A first partition 26 separates the inlet chamber 20 from the drift chamber 22 and a second partition 28 separates the drift chamber 22 from the outlet chamber 24. The first and second partitions 26, 28 are fluid-tight except for a first nanopore 30 in the first partition 26 and a second nanopore 32 in the second partition 28, which allow passage of liquid electrolyte and biomolecules suspended therein.
The partitions preferably comprise thin plates or solid-state membranes made of Si, SiN, $SiO_2$, or any other suitable material. The thickness d of the partitions 26, 28 in the region around the nanopores is preferably chosen close to the diameter D of the nanopores 30, 32. The nanopores may be produced by any suitable technique that allows obtaining a through-hole with a diameter of about 2 to 30 nm in a controlled manner, e.g. electron-beam lithography, possibly assisted by ion shooting, by ion beam sculpting, by etching either assisted or not by any preliminarily defect formation such as e.g. ion beam channelling. For examples for the composition of the partitions and the different nanopore fabrication techniques, the reader is referred to references [4-7].
The partitions 26, 28 are fixed to the receptacle 18 in a parallel fashion with respect to one another. The receptacle 18 may be made of any suitable material, inert with respect to the electrolyte solution and the biomolecules. Examples of possible materials are polytetrafluoroethylene (Teflon™), mica, polyethylene and glass. The internal surfaces of the receptacle 18 are preferably passivated with respect to the biomolecules.
The dimensions (length d, diameter D) of the nanopores 30, 32 may be chosen depending upon the application, which the device 16 is designed for. The nanopore diameter should be greater than that of the largest biomolecule among those the device 16 is dedicated to detect. In particular, the diameter of the nanopores 30, 32 may be 20 nm to detect proteins or other biomolecules having an effective diameter of up to 20 nm. If the samples contain only smaller biomolecules, the nanopore diameter is preferably chosen smaller, too: supposing the sample contains different protein species having all an effective diameter of at most 10 nm, the diameter of the nanopores is preferably selected in the range of 11 to 20 nm. In the present example, the nanopores 30, 32 of the first and the second partition have the same dimensions.
A first electrode 34 and a second electrode 36 are arranged in the inlet chamber 20 and the outlet chamber 24, respectively. The electrodes 34, 36 are connected to a measurement circuit comprising a voltage source 38 configured to apply a DC voltage (preferably in the range from 1 mV to 1 V, more preferably in the range from 1 mV to 500 mV) and a current meter 40 adapted for measurement of small currents (e.g. in the pA and nA range).
The first and second nanopores are arranged in facing relationship to one another, substantially at the intersections of the partitions 26, 28 with a (virtual) straight line extending from the first 34 to the second electrode 36.
Use of the device 16 for analyzing a sample of biomolecules is now explained in more detail with reference to Figs. 5 to 8. The chambers 20, 22, 24 of the device 16 are filled with an electrolyte solution compatible with the biomolecules of the sample to be analysed. The electrolyte solution could e.g. be normal saline or a buffer solution. The sample is introduced into the inlet chamber before, after or together with the portion of the electrolyte solution of that chamber. Any suitable injection process may be used. The device 16 is shown with an injection nozzle 42 to inject the sample into the inlet chamber 20, but this is only one of several possibilities. In the inlet chamber, the biomolecules of the different species of the sample form a more or less homogeneous mixture, illustrated in Fig. 5 as the cloud 44 (in practice, the cloud 44 may occupy the entire volume of the inlet chamber). When the sample is in place in the inlet chamber 20, a substantially constant voltage (in the range of e.g. 10 to 100 mV) is applied across the electrodes 36, 34 by switching on the voltage source. The voltage causes an ionic current to flow through the nanopores 30, 32 and the drift chamber 22. The current flowing in the electric circuit is measured using current meter 40 and recorded as a function of time. Under the action of the electric field, the biomolecules are caused to migrate from the inlet chamber 20 into the drift chamber 22 through the nanopore 30 of the first partition 26 (illustrated in Fig. 5). The biomolecules then move across the drift chamber 22 at a drift rate in accordance with their size-to-charge relation. Accordingly, each biomolecule species moves towards the second nanopore 32 with its individual speed. As they drift through the drift chamber, the biomolecules thus separate into different swarms 46, 48, 50, each swarm containing biomolecules of the same size-to-charge ratio (illustrated in Fig. 6). The swarms 46, 48, 50 of biomolecules arrive at the second nanopore 32 at different times. The electric field then causes the biomolecules to pass from the drift chamber 22 into the outlet chamber 24 through the nanopore 34 of the second partition 28 (illustrated in Fig. 7).

Fig. 8 shows a graph of the measured current as a function of time as it could be obtained when the experiment of Figs. 5 to 7 is carried out. (Fig. 8 does not show actually measured data.) Over time, the measured current shows significant drops 52, 54, 56, 58 of limited duration, between which the current returns to a substantially constant value. The appearance of the current curve is explained in the following. The voltage applied through the electrolyte solution is kept essentially constant in time. Each time a biomolecule passes through one of the nanopores 30, 32, it partially obstructs the nanopore and reduces the free cross section thereof, which is available for small ions ($Na^+$, $K^+$, $Cl^-$, or whichever other small ions might be present in the solution) to pass from one side of the partition to the other: as a consequence, there is an increase of the resistance between the electrodes 34, 36, which translates into a short drop of the measured current. The first drop 52 is in fact a series of individual short current drops, which are close together in time, caused by the biomolecules passing through the nanopore 30 of the first partition 26 (see Fig. 5). The later drops 54, 56 and 58 are caused by biomolecules passing the nanopore 32 of the second partition 28. Each swarm 46, 48, 50 causes a series of temporally close, short current drops as its molecules pass through the nanopore of the second partition. As each swarm arrives at a different time, the current drops 54, 56 and 58 show no overlap along the time axis and may be distinguished from one another.

The time of drift $\Delta T_1$, $\Delta T_2$, $\Delta T_3$ of each species is determined as the time interval between the first current drop 53 and the later current drop 54, 56 or 58. One preferably measures each time of drift from the middle To of the time interval occupied by the first current drop 52 to the middle ($T_1$, $T_2$ or $T_3$) of the later current drop 54, 56 or 58. The times of drift $\Delta T_1$, $\Delta T_2$, $\Delta T_3$ are used as fingerprints of the biomolecule species of the sample. Identification of the different species may be achieved by comparison of the measured times of drift and reference values previously measured using samples of well-known composition. If the distance between the nanopores 30 and 32 and the other relevant parameters (e.g. the composition of the electrolyte solution, its viscosity etc.) are precisely known (e.g. from calibration of the device), one may of course also identify the biomolecule species by comparison with values taken from the literature.

The electrical resistance $R_d$ of the drift chamber can be shown to be

$$R_d \sim 1/(\sigma\, r),$$

where "~" means "equal by the order of magnitude", r designates the nanopore radius and $\sigma$ designates the conductivity of the electrolyte solution. The electrical resistance Rp of a nanopore is

$$R_p \sim d/(\sigma\, r^2),$$

where d is the length of the nanopore (the thickness of the partition in the vicinity of the nanopore). If d ~ r, one finds $R_d \sim R_p$, which means that the voltage drop across the nanopore is of the same order of magnitude as the voltage drop across the drift chamber. It follows that the partial obstruction of a nanopore leads to a significant (detectable) drop in ionic current.

The time of drift $\Delta T$ of a biomolecule through the drift chamber can be described by the relation:

$$\Delta T \sim (L^3\, \rho\, \eta)/(r\, e\, n\, U),$$

where L designates the distance between the nanopores in the first and the second partition, $\rho$ the hydrodynamic radius of the biomolecule, $\eta$ the viscosity of the electrolyte solution, U the voltage across the device, e the elementary charge and n the charge number.

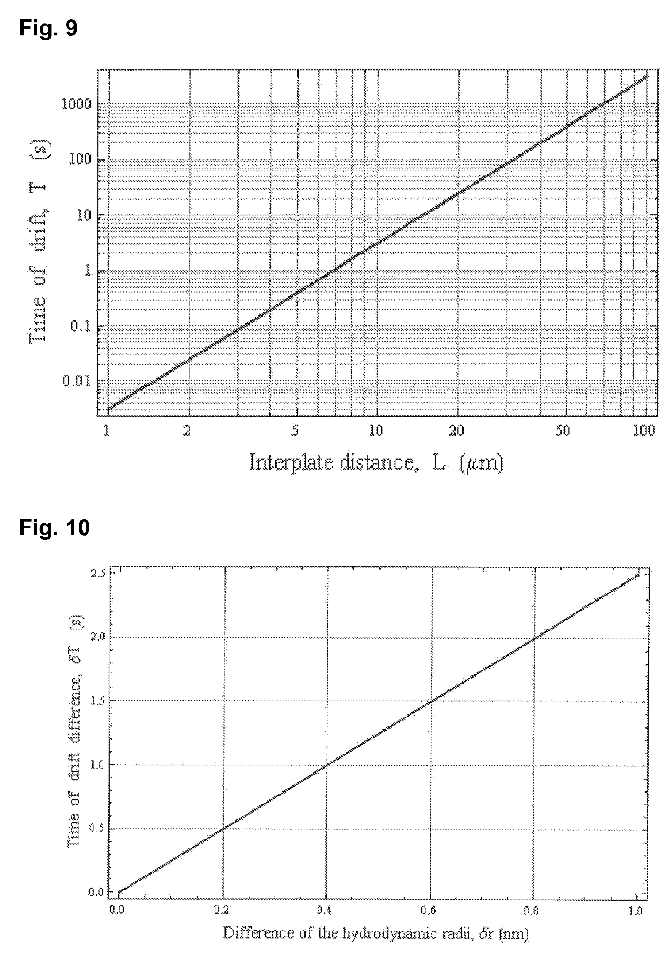

Assuming, as a first example, that the radius of both nanopores r = 20 nm, the hydrodynamic radius of the biomolecule $\rho$ = 10 nm and the number of charges n = 10 and that the biomolecule moves in a fluid with viscosity $\eta$ = $10^{-3}$ Pa s under the action of a voltage U = 0.1 V (these may be considered as typical values). The dependence of the time of drift $\Delta T$ upon the spacing L between the partitions is shown in Fig. 9. One can see that the drift takes between 10 ms for L = 1 $\mu$m to 1000 s (approximately 17 minutes) for L = 100 $\mu$m.

As a second example, consider two biomolecules differing from one another in size while all the other parameters are as in the first example hereinabove. Assuming L = 20 $\mu$m, one obtains the difference between the times of drift of these two molecules as a function of the difference between their radii as shown in Fig. 10. One can see that a difference in the hydrodynamic radius of only 0.1 nm gives rise to a time of drift difference of about 0.25 s, which can easily be resolved by electronics.

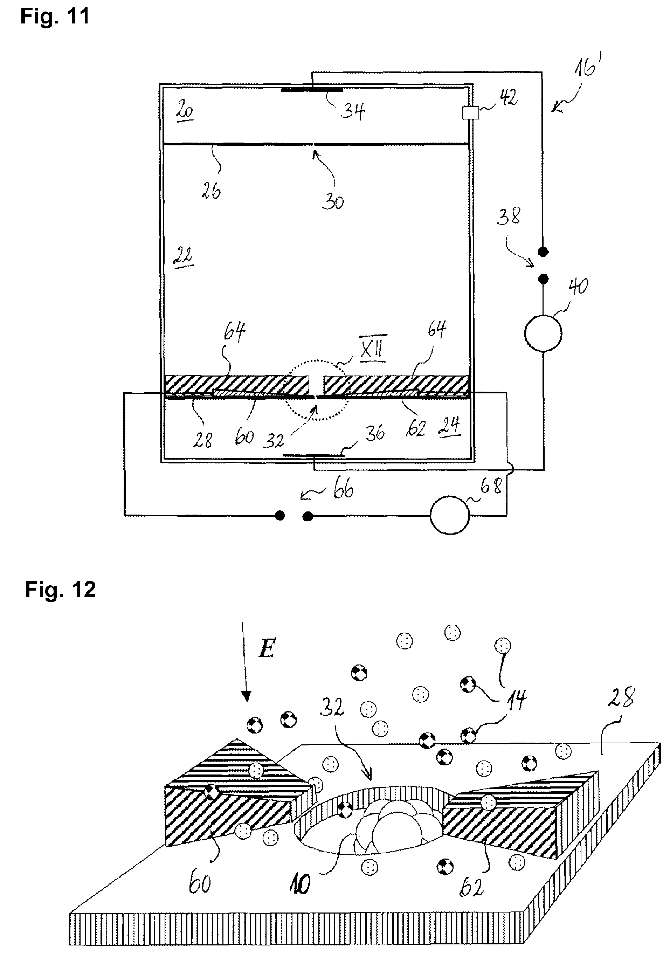

Fig 11 shows a variant 16' of the electrophoretic biomolecules separation device 16 of Figs. 3-7. Elements already encountered already in these figures have been given the same reference numbers in Fig. 11. These elements need not be discussed again as their function remains the same in the present embodiment of the invention. The device 16' comprises a pair of electrodes 60, 62 arranged in a plane perpendicular to the nanopore 32 in such a way as to form a "gating nanopore" (see [9]). The electrodes 60, 62 are preferably Au and/or Pt electrodes. Only the tips of the electrodes 60, 62 are exposed to the electrolyte; the remainder is covered with a protective layer 64 (e.g. made of $SiO_2$). The tips of the electrodes 60, 62 are separated by a distance corresponding approximately to the diameter of the nanopore 32. Fig. 12 shows a 3D close-up schematic view of the nanopore 32 of Fig. 11. In Fig. 12, the protective layer 64 is not shown for sake of clarity of the drawing.

The electrodes 60, 62 are connected to a measurement circuit comprising a voltage source 66 configured to apply a voltage preferably in the range from 1 mV to 1 V (more preferably in the range from 1 mV to 500 mV) and a current meter 68 adapted for measurement of small currents (e.g. in the pA and nA range). For the fabrication of such nanopore and electrodes, the reader is referred to reference [9].

The measurements may be carried out as with device 16 previously described. In addition to measuring the (longitdinal) current flowing between the electrodes 34 and 36, one may now also measure the (transversal) current flowing between the electrodes 60 and 62. Unlike the longitudinal current, which drops each time a biomolecule 10 translocates through the nanopore 32, the transversal current considerably increases in these situations. The transversal current may thus be recorded as a function of time. The drops of the longitudinal current may be correlated with the jumps of the transversal current e.g. to eliminate measurement artifacts and/or to increase measurement reliability. The transversal current could furthermore help to characterize the biomolecules passing though the nanopore 32.

It should be noted that the first nanopore 30 could be implemented as a gating nanopore in addition or as an alternative to the second nanopore 32.

**References**

[1] Borsdorf, H. & Eiceman, G. A. Ion mobility spectrometry: Principles and applications. Applied Spectroscopy Reviews 41, 323-375. (2006)

[2] Schriemer, D. C. & Li, L. Laser-induced surface ionization in a time-of-flight mass spectrometer. Review of Scientific Instruments, 66(1, Pt. 1), 55-62 (1995)

[3] Yang, M. & Reilly, J. P. A reflectron mass spectrometer with UV laser-induced surface ionization. International Journal of Mass Spectrometry and Ion Processes 75, 209-219 (1987)

[4] Dekker, C. Solid-state nanopores. Nature Nanotechnology 2, 209-215 (2007)

[5] Han, A. et al. Label-free detection of single protein molecules and protein-protein interactions using synthetic nanopores. Anal. Chem. 80, 4651-4658 (2008)

[6] Smeets, R. M. M. et al. Salt dependence of ion transport and DNA translocation through solid-state nanopores. Nano Letters 6, 89-95 (2006)

[7] Skinner, G. M., van den Hout, M., Broekmans, O., Dekker, C. & Dekker, N. H. Distinguishing Single- and Double-Stranded Nucleic Acid Molecules Using Solid-State Nanopores. Nano Letters 9, 2953-2960 (2009)

[8] van Dorp, S., Keyser, U. F., Dekker, N. H., Dekker, C. & Lemay, S. G. Origin of the electrophoretic force on DNA in solid-state nanopores. Nature Physics 5, 347-351 (2009)

[9] Taniguchi M, et al. Fabrication of the gating nanopore device. Applied Physics Letters 95, 123701 (2009)

**List of reference signs:**

| | |
|---|---|
| 10 | Biomolecule |
| 12 | Nanopore |
| 14 | Small ion |
| 16 | electrophoretic biomolecules separation device |
| 18 | Receptacle |
| 20 | Inlet chamber |
| 22 | Drift chamber |
| 24 | Outlet chamber |
| 26 | First partition |
| 28 | Second partition |
| 30 | First nanopore |
| 32 | Second nanopore |
| 34 | First electrode |
| 36 | Second electrode |
| 38 | Voltage source |
| 40 | Current meter |
| 42 | Injection nozzle |
| 44 | Cloud |
| 46-50 | Swarms of biomolecules |

| 52 | Initial current drop |
|---|---|
| 54-58 | Subsequent current drops |
| 60, 62 | Transversal electrode |
| 64 | Protective layer |
| 66 | Voltage source |
| 68 | Current source |
| D | Nanopore diameter |
| d | Nanopore length |

**Claims**

1. Device for electrophoretic separation of biomolecules, comprising an inlet chamber, a drift chamber and an outlet chamber, a first partition between the inlet chamber and the drift chamber, said first partition having therein at least one nanopore allowing fluid communication and passage of biomolecules between said inlet chamber and said drift chamber, a second partition between the drift chamber and the outlet chamber, said second partition having therein at least one nanopore allowing fluid communication and passage of biomolecules between said drift chamber and said outlet chamber; a first and a second electrode associated with said inlet chamber and said outlet chamber, respectively, for generation of an ionic current through said nanopores and said drift chamber.

2. The device according to claim 1, wherein said nanopores have a diameter adapted to the size of said biomolecules in such a way that the free cross section of said nanopores is substantially reduced when said biomolecules pass there through.

3. The device according to claim 1 or 2, wherein the cross section diameter of said nanopores is selected in the range from 2 to 50 nm, preferably in the range from 10 to 30 nm.

4. The device according to any one of claims 1 to 3, wherein the cross section diameter of the at least one nanopore in said first partition is selected in the range of 1 to 4 times, more preferably in the range of 1 to 3 times, still more preferably in the range of 1 to 2 times, the cross section diameter of the at least one nanopore in said second partition.

5. The device according to any one of claims 1 to 4, wherein the channel length of said nanopores is comprised in the range from 0.5 to 5 times the cross section diameter of said nanopores.

6. The device according to any one of claims 1 to 5, wherein said first and/or said second partition comprises a membrane, e.g. a solid-state membrane, in which said at least one nanopore is arranged.

7. The device according to any one of claims 1 to 6, wherein the volume of said inlet chamber is smaller, preferably at least 5 to 10 times smaller, than the volume of said drift chamber.

8. The device according to any one of claims 1 to 7, comprising an injection system for injecting a sample containing biomolecules into said inlet chamber.

9. The device according to any one of claims 1 to 8, wherein said chambers contain an electrolyte solution, such as, e.g. normal saline or a buffer solution.

10. The device according to any one of claims 1 to 9, wherein the internal surfaces of said inlet chamber, said drift chamber and said outlet chamber are passivated.

11. The device according to any one of claims 1 to 10, wherein said partitions are arranged substantially parallel to one another.

12. The device according to any one of claims 1 to 11, wherein said at least one nanopore of said first partition is arranged in facing relationship with said at least one nanopore of said second partition.

13. The device according to any one of claims 1 to 12, comprising a measurement circuit for applying a voltage across said first and second electrodes and to record resistance and/or current between said first and second electrodes as a function of time.

14. The device according to any one of claims 1 to 13, wherein said drift chamber is subdivided into at least two sections with one or more further partitions, each of said partitions comprising at least one nanopore allowing fluid communication and passage of biomolecules between neighboring sections.

15. Method for analyzing a sample containing biomolecules comprising:

   providing a device as claimed in any one of claims 1 to 14, the chambers of said device containing an electrolyte solution compatible with said the biomolecules of said sample;
   introducing said sample into said inlet chamber;
   applying a voltage across said electrodes to cause an ionic current to flow through said nanopores and said drift chamber and said biomolecules to migrate from said inlet chamber into said drift chamber through said at least one nanopore of said first partition, to move across said drift chamber in accordance with a size-to-charge relation of said biomolecules, and to migrate from said drift chamber into said outlet chamber through said at least one nanopore of said second partition;
recording a resistance and/or current between said first and second electrodes as a function of time.

## Fig. 1 (Prior Art)

## Fig. 2 (Prior Art)

**Fig. 3**

**Fig. 4**

**Fig. 5**

**Fig. 6**

**Fig. 7**

**Fig. 8**

**Fig. 9**

Interplate distance, L (μm)

**Fig. 10**

Difference of the hydrodynamic radii, δr (nm)

**Fig. 11**

**Fig. 12**

Europäisches
Patentamt

European
Patent Office

Office européen
des brevets

## EUROPEAN SEARCH REPORT

Application Number

EP 09 17 2950

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FU JIANPING ET AL: "Nanofilter array chip for fast gel-free biomolecule separation" APPLIED PHYSICS LETTERS, AIP, AMERICAN INSTITUTE OF PHYSICS, MELVILLE, NY, US, vol. 87, no. 26, 22 December 2005 (2005-12-22), pages 263902-263902, XP012077128 ISSN: 0003-6951 * the whole document * | 1-15 | INV. C12Q1/68 G01N27/447 G01N33/487 |
| X  A | US 2004/038260 A1 (MARTIN FRANCIS J [CA] ET AL) 26 February 2004 (2004-02-26) * paragraphs [0007], [0028] * * paragraph [0056] - paragraph [0063]; figures 8A,B * | 1-6,8-9, 11-14  15 | |
| E | WO 2010/007537 A1 (KONINKL PHILIPS ELECTRONICS NV [NL]; VAN DER ZAAG PIETER J [NL]; VAN D) 21 January 2010 (2010-01-21) * page 10, line 28 - page 12, line 5; figure 1 * | 1-2,8-9, 11-13 | |
| X  A | EP 1 645 628 A1 (AGILENT TECHNOLOGIES INC [US]) 12 April 2006 (2006-04-12)  * paragraphs [0028], [0072], [0078], [0079], [0098]; figure 4 * | 1-3,5-6, 8-9, 11-13  15 | TECHNICAL FIELDS SEARCHED (IPC) C12Q G01N |

The present search report has been drawn up for all claims

| Place of search | Date of completion of the search | Examiner |
|---|---|---|
| Munich | 19 March 2010 | Komenda, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 09 17 2950

This annex lists the patent family members relating to the patent documents cited in the above-mentioned European search report.
The members are as contained in the European Patent Office EDP file on
The European Patent Office is in no way liable for these particulars which are merely given for the purpose of information.

19-03-2010

| Patent document cited in search report | | Publication date | Patent family member(s) | | Publication date |
|---|---|---|---|---|---|
| US 2004038260 | A1 | 26-02-2004 | NONE | | |
| WO 2010007537 | A1 | 21-01-2010 | NONE | | |
| EP 1645628 | A1 | 12-04-2006 | JP 2006113057 A | | 27-04-2006 |
| | | | US 2006073489 A1 | | 06-04-2006 |

For more details about this annex : see Official Journal of the European Patent Office, No. 12/82

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **Borsdorf, H. ; Eiceman, G. A.** Ion mobility spectrometry: Principles and applications. *Applied Spectroscopy Reviews,* 2006, vol. 41, 323-375
- **Schriemer, D. C. ; Li, L.** Laser-induced surface ionization in a time-of-flight mass spectrometer. *Review of Scientific Instruments,* 1995, vol. 66, 55-62
- **Yang, M. ; Reilly, J. P.** A reflectron mass spectrometer with UV laser-induced surface ionization. *International Journal of Mass Spectrometry and Ion Processes,* 1987, vol. 75, 209-219
- **Dekker, C.** Solid-state nanopores. *Nature Nanotechnology,* 2007, vol. 2, 209-215
- **Han, A. et al.** Label-free detection of single protein molecules and protein-protein interactions using synthetic nanopores. *Anal. Chem.,* 2008, vol. 80, 4651-4658

- **Smeets, R. M. M. et al.** Salt dependence of ion transport and DNA translocation through solid-state nanopores. *Nano Letters,* 2006, vol. 6, 89-95
- **Skinner, G. M. ; van den Hout, M. ; Broekmans, O. ; Dekker, C. ; Dekker, N. H.** Distinguishing Single- and Double-Stranded Nucleic Acid Molecules Using Solid-State Nanopores. *Nano Letters,* 2009, vol. 9, 2953-2960
- **van Dorp, S. ; Keyser, U. F. ; Dekker, N. H. ; Dekker, C. ; Lemay, S. G.** Origin of the electrophoretic force on DNA in solid-state nanopores. *Nature Physics,* 2009, vol. 5, 347-351
- **Taniguchi M et al.** Fabrication of the gating nanopore device. *Applied Physics Letters,* 2009, vol. 95, 123701